# EUROPEAN PATENT APPLICATION

(11) **EP 4 206 671 A1**
(43) Date of publication of application: **05.07.2023**
(21) Application number: 21872527.3
(22) Date of filing: 24.09.2021
(51) Int. Cl.: G01N 33/50, G01N 33/53, G01N 33/543, G01N 33/545

(54) **FERRITIN MEASURING REAGENT**

(30) Priority: 25.09.2020 JP 2020161136
(71) Applicant: Denka Company Limited, Tokyo 103-8338 (JP)
(72) Inventor: HIRAKAWA Yuki, Gosen-shi, Niigata 959-1695 (JP); TAKAHASHI Takamichi, Gosen-shi, Niigata 959-1695 (JP); TACHIBANA Ritsuko, Gosen-shi, Niigata 959-1695 (JP)
(74) Representative: Kordel, Mattias
(86) International application number: PCT/JP2021/034934
(87) International publication number: WO 2022/065398

(57) **Abstract**

Provided are a measuring reagent and a method for accurately measuring a high concentration of ferritin. The ferritin measuring reagent and the method for measuring ferritin are characterized by using liver-derived ferritin as a standard-solution raw material.

## Description

### Technical Field

The present invention relates to a test kit for detecting ferritin and a method for detecting ferritin.

### Background Art

Ferritin is an about 480-kDa intracellular iron storage complex protein containing 24 polypeptide subunits. This iron storage complex protein, found at high concentrations in serum, can contain as many as 4,500 atoms of iron ions (Fe³⁺) in the iron hydroxide core.

Since ferritin is present in many organs such as liver, spleen, and placenta, quantifying this ferritin is useful for diagnosing iron deficiency anemia, iron overload, and the like.

Further, in the case of leukemia and malignant tumors such as other solid cancers, the serum ferritin level increases regardless of the amount of iron stored in the body. Hence, quantifying ferritin is also useful for diagnosis and monitoring of the treatment of malignant tumors.

Conventionally, a radioimmunoassay has been mainly used for measuring the concentration of ferritin. However, since this method uses radioisotopes, an enzyme immunoassay method and an immunoassay using latex agglutination (latex nephelometry, counting immunoassay method, etc.) have been increasingly used in recent years.

In particular, the immunoassay using latex agglutination is widely used because this method enables simpler and faster measurement compared with other methods (see Patent Literature 1).

Capability of measuring a low concentration of ferritin has been conventionally required. However, a reference value for initiating the treatment of post-transfusion iron overload is "serum ferritin level of 1,000 ng/mL or more", and thus measuring a high concentration of ferritin has also been required in recent years.

In clinical examinations, about 5% of specimens have serum ferritin levels exceeding 1,000 ng/mL. Since ferritin measuring reagents that are used for a conventional immunoassay using latex agglutination have been unable to measure a high concentration of ferritin exceeding 1,000 ng/mL, these specimens have been diluted for re-measurement.

### Citation List

### Patent Literature

Patent Literature 1: JP Patent Publication (Kokai) No. 10-115615A(1998)

### Summary of Invention

### Technical Problem

Since conventional ferritin measuring reagents have been unable to measure a high concentration of ferritin, specimens have been diluted for re-measurement.

If a high concentration of ferritin can be measured, there is no need to dilute a specimen for re-measurement, and this is useful in terms of cost performance and shortening TAT (Turn Around Time: time from the arrival of a specimen to the acquisition of the test results).

However, it has been difficult to accurately measure a high concentration of ferritin.

An object of the present invention is to provide a measuring reagent and a method for accurately measuring a high concentration of ferritin.

### Solution to Problem

In view of the above problems, the present inventors have discovered that a high concentration of ferritin can be accurately measured using liver-derived ferritin as a standard-solution raw material, and the present invention has been completed based on these findings.

Specifically, the present invention is as follows.
[1] A ferritin measuring reagent, wherein liver-derived ferritin is used as a standard-solution raw material.
[2] The ferritin measuring reagent according to [1], which is a reagent for an immunoagglutination method.
[3] The ferritin measuring reagent according to [2], which is a reagent for a latex agglutination method.
[4] The ferritin measuring reagent according to any one of [1] to [3], comprising insoluble carrier particles sensitized with an anti-ferritin antibody and a standard solution prepared using liver-derived ferritin as a raw material.
[5] The ferritin measuring reagent according to any one of [1] to [4], comprising the standard solution having at least one ferritin concentration between 1,001 ng/mL and 2,000 ng/mL.
[6] A method for measuring ferritin, comprising using liver-derived ferritin as a standard-solution raw material.
[7] The method for measuring ferritin according to [6], which is an immunoagglutination method.
[8] The method for measuring ferritin according to [7], which is a latex agglutination method.
[9] The method for measuring ferritin according to any one of [6] to [8], comprising mixing ferritin in a test sample with insoluble carrier particles sensitized with an anti-ferritin antibody in a solution, measuring an absorbance based on an agglutination reaction due to an antigen-antibody reaction, applying the absorbance to a calibration curve, so as to quantify ferritin, wherein the calibration curve is prepared using a standard solution prepared using liver-derived ferritin as a raw material.
[10] The method for measuring ferritin according to any one of [6] to [9], wherein the standard solution has at least one ferritin concentration between 1,001 ng/mL and 2,000 ng/mL.

This description includes the contents as disclosed in the Japanese Patent Application No. 2020-161136, which is a priority document of the present application.

### Advantageous Effects of Invention

By the use of the ferritin measuring reagent of the present invention, a high concentration of ferritin can be accurately measured.

Moreover, the ferritin measuring reagent of the present invention needs no dilution of a specimen for re-measurement, and thus is useful in terms of cost performance and shortening TAT (Turn Around Time: time from the arrival of a specimen to the acquisition of the test results).

### Brief Description of Drawings

[Figure 1] Figure 1 depicts the results of measuring specimens in a low-concentration region using a standard solution prepared using liver-derived ferritin.
[Figure 2] Figure 2 depicts the results of measuring specimens in a low-concentration region using a standard solution prepared using spleen-derived ferritin.
[Figure 3] Figure 3 depicts the results of measuring specimens in a low-concentration region using a standard solution prepared using placenta-derived ferritin.
[Figure 4] Figure 4 depicts the results of measuring specimens in a high-concentration region using a standard solution prepared using liver-derived ferritin.
[Figure 5] Figure 5 depicts the results of measuring specimens in a high-concentration region using a standard solution prepared using spleen-derived ferritin.
[Figure 6] Figure 6 depicts the results of measuring specimens in a high-concentration region using a standard solution prepared using placenta-derived ferritin.

### Description of Embodiments

Hereinafter, the method of the present invention will be described. Note that "%" in this description means weight per volume % (w/v%) unless otherwise specified.

The present invention is characterized by using liver-derived ferritin as a standard-solution raw material in the measurement performed using a technique for immunoanalysis of ferritin.

The immunoanalysis technique itself is well known. The immunoanalysis method, to which the method of the present invention is applied, is an immunoagglutination method using insoluble carrier particles. In the present invention, the immunoagglutination method is well known as a method that involves causing an antigen-antibody reaction to take place between particles sensitized (bound) with an antibody and an antigen in a biological sample to clump together in a solution, and optically detecting the agglutination. A turbidimetric technique or a chromogenic technique is preferably used for detection. The immunoagglutination method is also referred to as an immunonephelometry. For example, clumps are irradiated with light in the visible to near-infrared region, for example, usually 300 nm to 1,000 nm, preferably 500 nm to 900 nm from the outside of the cell for detection of a change in absorbance or a change in the intensity of scattered light, and thus the degree of agglutination of the sensitized particles is measured. A method for detecting the agglutination of sensitized particles by the immunoagglutination method is well known, and also in the present invention, a well-known method such as a method for detecting absorbance, light scattering or the like due to agglutination of sensitized particles can be used.

Insoluble carrier particles to be used in an immunoagglutination method are not particularly limited, and may be well-known particles conventionally used for immunoanalysis reagents. Examples of insoluble carrier particles include latex particles such as polyethylene and polystyrene, alumina particles, silica particles, gold colloids, and magnetic particles. Among these insoluble carriers, latex particles, particularly polystyrene latex particles, are preferably used. Polystyrene latex particles are particularly preferably used as the latex particles. An immunoagglutination method using latex particles is referred to as a latex agglutination method. The size of insoluble carrier particles such as latex particles is not particularly limited, but the particle size preferably ranges from 30 nm to 600 nm.

An antibody against ferritin (anti-ferritin antibody) or an antigen-binding fragment thereof is immobilized on the above-mentioned latex particles. The antibody against ferritin can be prepared by a known method, or a commercially available one may be used. Examples of the antigen-binding fragment include immunoglobulin fragments such as Fab and F(ab')₂, or recombinant antibodies such as scFv, dsFv, diabody, and minibody expressed as recombinants. Methods for preparing these fragments are well known in the art. Immobilization methods are also well known and are performed by well-known methods such as physical adsorption or covalent bonding. The amount of the antibody to be immobilized is not particularly limited, but the amount of the antibody ranges from preferably 0.01 mg/mL to 2.0 mg/mL in a latex suspension. When the obtained suspension of sensitized particles and a test sample are mixed, the substance (antigen) to be measured contained in the test sample causes the sensitized particles to clump together, and the absorbance of the suspension of the sensitized particles changes. At this time, the concentration of the sensitized particles in the suspension is not particularly limited, but ranges from preferably 0.01% to 0.5%. For example, a specimen solution in a volume ranging from 5 µL to 20 µL is subjected to measurement of amounts of changes (endpoint method) or rates of changes (rate method) in absorbance. A plurality of standard samples containing the antigen to be measured at various known concentrations are prepared, and these samples are subjected to measurement of the amounts of changes or the rates of changes in absorbance by the above method. A calibration curve is drawn by plotting the concentrations of the antigen to be measured in the standard samples on the horizontal axis, and amounts of changes or rates of changes in absorbance measured on the vertical axis. For unknown test samples, amounts of changes or rates of changes in absorbance are measured by the same method, the measurement results are applied to a calibration curve prepared using a ferritin standard solution for comparison, and thus the antigen in the test sample(s) can be quantified.

The reaction temperature when an agglutination reaction is performed ranges from appropriately about 30°C to 40°C, and is most preferably 37°C. The reaction time may be about 2 to 10 minutes.

Various automatic devices for performing such an immunoagglutination method are commercially available, and can be easily and simply performed using a commercially available automatic device for the immunoagglutination method.

The substance to be measured in the present invention is ferritin.

A test sample is not particularly limited as long as it contains ferritin, and examples thereof include body fluids such as blood, serum, plasma, urine, stool, saliva, interstitial fluids, spinal fluids, and swabs, or dilutions thereof. Blood, serum, plasma, urine, stool, spinal fluids or dilutions thereof are preferred.

In the method of the present invention, a specimen is preferably diluted 5 to 20 times, preferably 10 to 20 times for measurement. Dilution may be performed using physiological saline, a buffer solution, purified water or the like. For example, 5 to 10 µL, and preferably 7 µL of a specimen may be diluted with 100 µL of a buffer solution for use. The thus diluted specimen may be mixed with 20 µL to 100 µL, preferably 25 µL to 75 µL, and more preferably 50 µL of a latex suspension for measurement.

As described above, in the present invention, human liver-derived ferritin is used as a standard-solution raw material for measurement of ferritin. The standard solution may be prepared in a dilution series between 0 ng/mL and 2,000 ng/mL, for example, and may be prepared every 100 ng/mL or every 200 ng/mL. The standard solution has at least one ferritin concentration between 1,001 ng/mL and 2,000 ng/mL. It is preferable to measure the standard solution at the same time as measuring ferritin in a specimen, or, the standard solution is measured in advance by the immunoagglutination method, a calibration curve is prepared, ferritin in a specimen is measured, and thus the ferritin concentration in the specimen can be found from the calibration curve based on the obtained absorbance.

The concentration region ranging from 0 ng/mL to 1,000 ng/mL is referred to as a low concentration region, and the concentration region exceeding 1,000 ng/mL is referred to as a high concentration region. According to the method of the present invention using liver-derived ferritin as a standard sample, ferritin in a specimen can be accurately quantified even in the high concentration region.

As described above, in the method of the present invention, a specimen is diluted 5 to 20 times for measurement. Such dilution of a specimen is generally performed in-device with an automatic analyzer. With a conventional method, a specimen having a serum ferritin level exceeding 1,000 ng/mL cannot be measured even if diluted, and re-dilution is required for measurement. However, according to the method of the present invention using liver-derived ferritin as a raw material of a standard solution, a specimen having a serum ferritin level exceeding 1,000 ng/mL can be measured without re-dilution.

A blank sample to be used for immunoanalysis is not particularly limited as long as it cannot contain ferritin; that is a substance to be measured, and is preferably purified water, a physiological saline solution, a buffer solution, a negative specimen or a diluted product thereof.

As described in the following Examples, when a polyoxyethylene alkyl ether surfactant having 8 to 14 carbon atoms of the alkyl group is present in a reaction and/or measurement system, a non-specific reaction is suppressed. For example, a polyoxyethylene alkyl ether surfactant having 8 to 14 carbon atoms of the alkyl group may be contained in a liquid to be mixed with a specimen. Alternatively, such polyoxyethylene alkyl ether surfactant may be contained in a latex particle suspension. The specificity is more significantly improved as compared with a case where the polyoxyethylene alkyl ether surfactant having 8 to 14 carbon atoms of the alkyl group is not present. Therefore, the use of the method of the present invention makes it possible to improve the reagent performance of a reagent with higher sensitivity, with which a non-specific reaction is likely to occur, as compared with conventional cases. The concentration of the polyoxyethylene alkyl ether surfactant in a reaction and/or measurement system ranges from 0.0001% to 2%, preferably 0.005% to 1%.

The present invention encompasses a ferritin measuring reagent characterized by using liver-derived ferritin as a standard-solution raw material. The measuring reagent contains at least insoluble carrier particles sensitized with an anti-ferritin antibody and a standard solution prepared using liver-derived ferritin as a raw material. The measuring reagent is also referred to as a measuring kit.

Furthermore, whether the standard-solution raw material is liver-derived ferritin or ferritin derived from another organ can be confirmed by analysis of contaminating organ-specific substances. Examples of an analysis method for organ-specific substances, which can be used herein, include proteome analysis and metabolome analysis.

For example, proteome analysis is a method for comprehensively identifying a protein by fragmenting a trace amount of a protein mixed in a standard solution into a peptide by protease, and then searching a database for the amino acid sequence of the peptide obtained by a mass spectrometer. Further, metabolome analysis is a method for comprehensively identifying a trace amount of metabolites mixed in a standard solution with a mass spectrometer. If the identified proteins or metabolites include proteins or metabolites that are specifically expressed in the liver, the ferritin is considered to be derived from the liver.

### Examples

The present invention will be specifically described with reference to the following Examples, but the present invention is not limited to these Examples.

### (1) Preparation of Reagent

Using an antibody against ferritin, a measuring reagent was prepared by an immunoagglutination method as follows.

Sensitized particles carrying 0.04 mg of anti-ferritin antibody per 1 mL of a polystyrene latex suspension were suspended in a buffer solution (Tris, pH 8.5) in such a manner that the content was 0.09%, thereby preparing the latex suspension.

### (2) Preparation of standard solution

Standard solutions to be used for preparing a calibration curve were prepared using ferritin.

Liver-derived ferritin (commercially available from SCRIPPS LABORATORIES) was added to a buffer solution (Hepes, pH 7.5) to prepare a standard solution A (Example 1). As Comparative Examples 1 and 2, ferritins derived from different organs were added to prepare standard solutions B and C.

**[Table 1]**

| Example | Standard solution | Ferritin-derived organ |
|---|---|---|
| Example 1 | A | Liver |
| Comparative Example 1 | B | Spleen |
| Comparative Example 2 | C | Placenta |

### (3) Measurement by automatic analyzer

Automatic measurement was performed by the endpoint method using a Hitachi 7180 automatic analyzer.

Forty-eight (48) serum specimens were measured using the reagent described above. A buffer solution (100 µL, Hepes, pH 7.4) was added to 7.0 µL of a specimen solution, and then the mixture was stirred and mixed at 37°C. After the mixture was left to stand for 5 minutes, 50 µL of the latex suspension was added, and then the mixture was further stirred and mixed at 37°C. The agglutination reaction for about 5 minutes was measured as an amount of change in absorbance, and then the ferritin concentration of each specimen was calculated based on the calibration curve prepared using the standard solutions A to C.

### (4) Comparison between parameters with different photometric points

In order to compare the reactivity, specimens were each measured using two parameters with different photometric points.

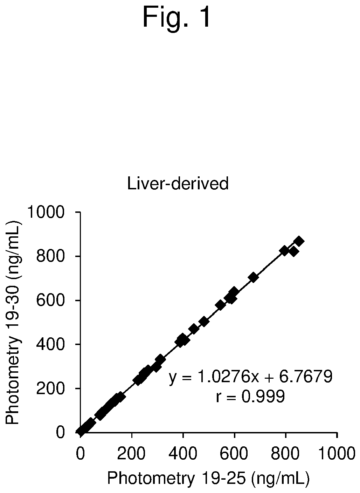

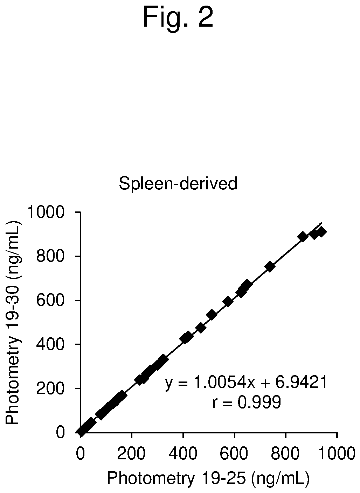

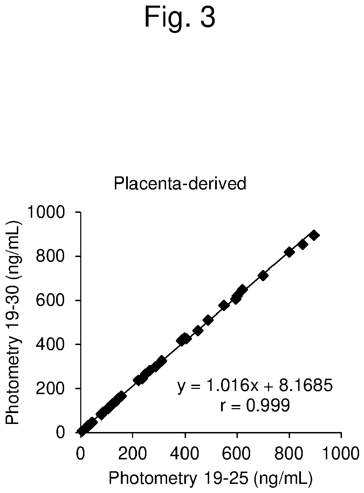

In the low concentration region (0 ng/mL to 1,000 ng/mL), when Figure 1 depicting the results of Example 1 was compared with Figures 2 and 3 depicting the results of Comparative Examples 1 and 2, the correlation coefficient was as good as 0.999 under all conditions, and no difference was confirmed due to different organs from which ferritins had been derived.

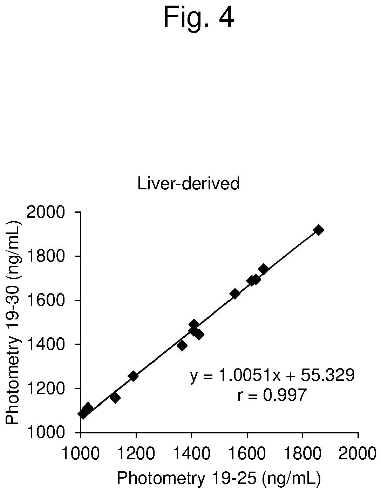

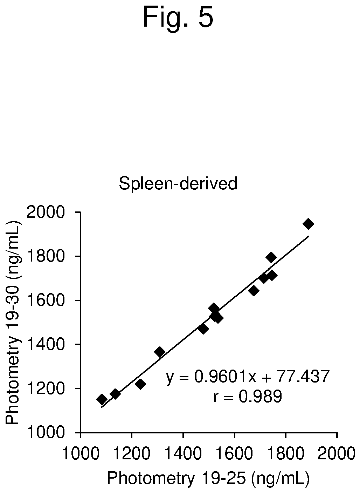

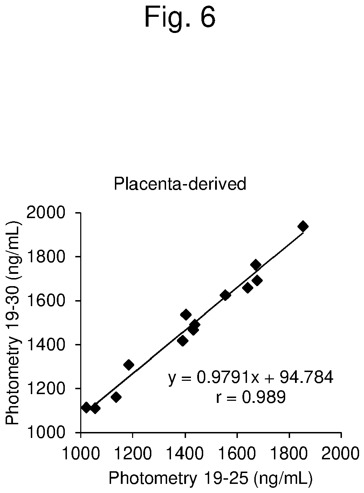

On the other hand, in the high concentration region (1,000 ng/mL to 2,000 ng/mL), when Figure 4 depicting the results of Example 1 was compared with Figures 5 and 6 depicting the results of Comparative Examples 1 and 2, the correlation coefficient was 0.997 in the case of using liver-derived ferritin as a standard-solution raw material, which was better than those of other conditions. Good correlation between different photometric points means that the measured values do not change even if the photometric points are varied, indicating that the time course of the standard solution and that of the specimen are similar. Therefore, it was demonstrated that a standard solution made of liver-derived ferritin as a raw material has reactivity similar to that of the specimen in the high concentration region.

### Industrial Applicability

Ferritin in a specimen can be measured by the method of the present invention.

All publications, patents and patent applications cited herein are incorporated herein by reference in their entirety.

## Claims

1. A ferritin measuring reagent, wherein liver-derived ferritin is used as a standard-solution raw material.

2. The ferritin measuring reagent according to claim 1, which is a reagent for an immunoagglutination method.

3. The ferritin measuring reagent according to claim 2, which is a reagent for a latex agglutination method.

4. The ferritin measuring reagent according to any one of claims 1 to 3, comprising insoluble carrier particles sensitized with an anti-ferritin antibody and a standard solution prepared using liver-derived ferritin as a raw material.

5. The ferritin measuring reagent according to any one of claims 1 to 4, comprising the standard solution having at least one ferritin concentration between 1,001 ng/mL and 2,000 ng/mL.

6. A method for measuring ferritin, comprising using liver-derived ferritin as a standard-solution raw material.

7. The method for measuring ferritin according to claim 6, which is an immunoagglutination method.

8. The method for measuring ferritin according to claim 7, which is a latex agglutination method.

9. The method for measuring ferritin according to any one of claims 6 to 8, comprising mixing ferritin in a test sample with insoluble carrier particles sensitized with an anti-ferritin antibody in a solution, measuring an absorbance based on an agglutination reaction due to an antigen-antibody reaction, applying the absorbance to a calibration curve, so as to quantify ferritin, wherein the calibration curve is prepared using a standard solution prepared using liver-derived ferritin as a raw material.

10. The method for measuring ferritin according to any one of claims 6 to 9, wherein the standard solution has at least one ferritin concentration between 1,001 ng/mL and 2,000 ng/mL.
